# EUROPÄISCHE PATENTANMELDUNG

(11) **EP 1 825 849 A1**
(43) Veröffentlichungstag der Anmeldung: **29.08.2007**
(21) Anmeldenummer: 07001087.1
(22) Anmeldetag: 10.07.2002
(51) Int. Cl.: A61K 9/70, A61K 31/4468

(54) **Transdermales therapeutisches System mit Fentanyl**

(30) Priorität: 24.08.2001 DE 10141650
(62) Teilanmeldung aus: 02762336.2
(71) Anmelder: LTS LOHMANN Therapie-Systeme AG, 56626 Andernach (DE)
(72) Erfinder: Müller, Walter, 56626, Andemach (DE); Hille, Thomas, 56567, Neuwied (DE)
(74) Vertreter: Isenbruck, Günter

(57) **Zusammenfassung**

Die Erfindung bezieht sich auf ein transdermales therapeutisches System (TTS) bestehend aus einer wirkstoffundurchlässigen Rückschicht, zumindest einer Fentanyl oder einen fentanylanalogen Wirkstoff enthaltenden Matrixschicht auf Basis von Polyacrylat und einer vor Gebrauch zu entfernenden Schutzschicht, das dadurch gekennzeichnet ist, daß das Polyacrylatpolymer selbstklebend und frei von Carboxylgruppen ist, für Fentanyl eine Sättigungslöslichkeit zwischen 3 und 20 Gewichtsprozenten, bevorzugt eine Sättigungslöslichkeit zwischen 4 und 12 und besonders bevorzugt eine Sättigungslöslichkeit zwischen 5 und 10 Gewichtsprozenten aufweist, und daß die wirkstoffhaltigen Schichten mindestens 80 Gewichtsprozent des eingearbeiteten Wirkstoffs in molekulardispers gelöster Form enthalten.

## Beschreibung

Fentanyl und fentanylanaloge Substanzen wie Sulfentanyl, Carfentanyl, Lofentanyl und Alfentanyl sind außerordentlich wirksame Analgetika. Das Erfordernis der nur geringen Dosierung und ihre physiko-chemischen Eigenschaften wie der n-Octanol-Wasser-Verteilungskoeffizient, Schmelzpunkt und das Molekulargewicht machen die transdermale Zufuhr dieser Substanzen in wirksamer Menge möglich und ihre pharmakokinetischen Eigenschaften wie die schnelle Metabolisierung und der relativ enge therapeutische Index die transdermale Zufuhr wünschenswert.

In der Tat ist seit einigen Jahren ein TTS mit Fentanyl als Wirkstoff auf dem Markt. Dieses System ist ein sogenanntes Reservoirsystem. Unter einem Reservoirsystem wird dabei ein System verstanden, das den Wirkstoff in einer flüssigen oder gelförmigen Zubereitung in einem aus einer undurchlässigen Folie, die als Rückschicht dient, und einer wirkstoffdurchlässigen Membran geformten Beutel enthält, wobei die Membran zusätzlich mit einer Kleberschicht zur Befestigung des Systems auf der Haut versehen ist. In diesem speziellen Fall ist Fentanyl in einem Gemisch aus Ethanol und Wasser gelöst. Weitere Einzelheiten dieses Systems können der US-Patentschrift 4,588,580 bzw. der DE-PS 35 26 339 entnommen werden, die beide eine detaillierte Beschreibung enthalten.

Reservoirsysteme haben allerdings den Nachteil, daß im Falle einer Undichtigkeit des Reservoirbeutels die wirkstoffhaltige Reservoirfüllung großflächig mit der Haut in Kontakt kommt und der Wirkstoff in zu hohen Dosen resorbiert wird. Dies ist speziell bei Fentanyl und seinen Derivaten sehr gefährlich, da eine Überdosierung sehr schnell zu Atemdepression und damit tödlichen Zwischenfällen führt. Mehrere solcher tödlichen bzw. fast tödlichen Zwischenfälle sind beschrieben in Clinical Pharmacokinet. 2000, 38(1), 59-89.

Aufgabe dieser Erfindung war es nun ein transdermales therapeutisches System mit Fentanyl bzw. Fentanylanalogen bereitzustellen, das dem Benutzer eine erhöhte Sicherheit gegen eine versehentliche Aufnahme von Überdosen bietet.

Dies gelingt erfindungsgemäß dadurch, daß statt des Reservoirsystems ein Matrixsystem eingesetzt wird, bei dem der Wirkstoff direkt in ein selbstklebendes Polyacrylat eingearbeitet wird und damit selbst bei einer Beschädigung des Systems nicht in einer größeren als durch das TTS gegebenen Fläche mit der Haut in Kontakt kommen kann. Der Wirkstoff ist in einem solchen System im allgemeinen ganz, jedoch zu mindestens 80 % in diesem Polymer molekulardispers gelöst, wobei die Sättigungslöslichkeit des Wirkstoffs im Polymer zwischen 3 und 20 Gewichtsprozenten liegt. Weiterhin hat es sich überraschenderweise gezeigt, daß bei der Verwendung von Polyacrylatklebern für die Herstellung von TTS mit Fentanyl und seinen Analogen nur Kleber ohne freie Carboxylgruppen geeignet sind.

Solche Matrixsysteme bestehen im einfachsten Fall aus einer Rückschicht, die für den Wirkstoff undurchlässig ist, einer selbstklebenden wirkstoffhaltigen Schicht und einer vor Gebrauch zu entfernenden Schutzschicht. In komplizierteren Ausführungen solcher Systeme schließt sich zusätzlich noch eine die Wirkstoffabgabe steuernde Membran an, die normalerweise noch mit einer Kleberschicht zur Befestigung des Systems auf der Haut versehen ist.

Die wirkstoffhaltigen Schichten eines solchen Matrixsystems gemäß dieser Erfindung bestehen aus Polyacrylaten. Da freie funktionelle Gruppen die Sättigungslöslichkeit von Fentanyl und seinen Derivaten in Polyacrylatklebern über den bevorzugten Bereich erhöhen, sind am besten solche Polyacrylatkleber geeignet, die über keine freien funktionellen Gruppen verfügen und lediglich aus Estern der Acryl- und/oder Methaycrylsäure und gegebenenfalls sonstigen Vinylverbindungen ohne freie funktionelle Gruppen wie Vinylacetat hergestellt werden. Allerdings können bei der Klebersynthese Monomere mit freien Hydroxylgruppen wie 2-Hydroxyethylacrylat oder 2-Hydroxyethylmethacrylat bis zu einem Anteil von 20 Gew.-% toleriert werden. Polyacrylate werden durch radikalische Polymerisation unter Verwendung von Acryl- und/oder Methacrylsäurederivaten hergestellt. Solche Derivate sind z.B. Ester. Beispielhaft für solche Derivate seien Acryl- und Methacry!säurederivate genannt, insbesondere Ester von Alkoholen mit 1 bis 8 C-Atomen, die gegebenenfalls eine Hydroxylgruppe enthalten, wie 2-Ethylhexylacrylat, n-Octylacrylat, Propylacrylat, n-oder iso-Butylacrylat, 2-Hydroxyethylacrylat und Dimethylaminoethylacrylat bzw. die entsprechenden Methacrylate. Zusätzlich können auch andere polymerisierbare Vinylverbindungen ohne freie funktionelle Gruppen wie z.B. Vinylacetat mitverwendet werden, z. B. in Mengen von bis zu 50 Gew.-%. Die so hergestellten Polymere werden auch als statistische Copolymere bezeichnet, da allein die Mengenverteilung der eingesetzten Monomeren und der Zufall über die Zusammensetzung der Polymerketten entscheidet.

Enthalten die Polymere freie Hydroxylgruppen, besteht die Möglichkeit, die Polymerketten zusätzlich durch mehrwertige Kationen wie Al³⁺ oder Ti⁴⁺ oder reaktive Substanzen wie Melamin zu vernetzen. Man macht von dieser Möglichkeit Gebrauch um das Molekulargewicht zu erhöhen und damit die Kohäsion der Polymere zu verbessern. Die Möglichkeit der Quervernetzung von Polyacrylaten, insbesondere von Polyacrylatklebern, ist besonders wertvoll, wenn die weichmachende Wirkung des in den Polymeren gelösten Wirkstoffs bzw. die weichmachende Wirkung von anderen Hilfsstoffen kompensiert werden muß. Der Kleber wird gewöhnlich in Form einer Lösung verwendet. Als Lösungsmittel dienen z. B. Ethylacetat, Hexan oder Heptan, Ethanol oder deren Mischungen. Diese werden während der Herstellung des TTS entfernt.

In Tabelle 1 sind die Ergebnisse von Permeationsstudien gezeigt, die mit einem Kleber mit und einem Kleber ohne freie Carboxylgruppen (jedoch ohne Hydroxylgruppen) erreicht worden sind. In beide Kleber wurde der Wirkstoff in einer Konzentration von 5 Gewichtsprozenten eingearbeitet. Die Permeationsstudie wurde mittels dem Fachmann bekannter Franz-Diffusionszellen und Verwendung von menschlicher Haut durchgeführt.

**Tabelle 1: Ergebnisse von Permeationsstudien mit Klebern mit und ohne freie Carboxylgruppen**

| Formulierung | Kumulierte Menge an permeiertem Wirkstoff [µg/cm²] Mittelwert von n = 3* | | | | |
|---|---|---|---|---|---|
| | 4 h | 8 h | 24 h | 48 h | 72 h |
| 1 | 0,00 | 0,00 | 0,44 | 1,71 | 3,51 |
| 2 | 0,0 | 0,2 | 4,0 | 14,7 | 28,24 |

| | | | | | |
|---|---|---|---|---|---|
| * verwendete Haut: weibl. Unterleibshaut | | | | | |

- Formul. 1:: Polyacrylatkleber mit 4,8 Gew.-% freien Carboxylgruppen
- Formul. 2:: neutraler Polyacrylatkleber ohne freie Carboxylgruppen aber mit 5,2 Gew.-% freien Hydroxylgruppen

Die Ergebnisse zeigen, daß ein neutraler Kleber ohne freie Carboxylgruppen einem carboxylgruppenhaltigen Kleber bezüglich der erreichbaren Permeationsraten deutlich überlegen ist.

Eine wichtige Eigenschaft jedes wirkstoffhaltigen Polymers in der TTS-Technologie ist die Sättigungslöslichkeit des gewählten Polymers für den jeweiligen Wirkstoff. Dieser Parameter ist deshalb wichtig, weil die thermodynamische Aktivität des Wirkstoffs in der Matrix nicht von der absolut gelösten Menge des Wirkstoffs, sondern vielmehr vom Verhältnis der tatsächlichen Konzentration zu der Sättigungskonzentration abhängt. Da sich der Wirkstoff bei der Applikation des TTS auf die Haut in die Haut verteilen muß und sich dabei nicht Konzentrationen, sondern Aktivitäten angleichen, ist es zum Erreichen einer möglichst hohen Permeationsrate wichtig, die thermodynamische Aktivität des Wirkstoffs im TTS möglichst hoch zu wählen. Dies bedeutet, daß die Löslichkeit des Wirkstoffs in den wirkstoffhaltigen Teilen des TTS nicht zu hoch sein darf, da ansonsten die Wirkstoffkonzentration im TTS recht hoch sein muß um eine genügend hohe thermodynamische Aktivität zu erreichen. Dies ist unvorteilhaft, wenn der Wirkstoff in der hohen Konzentration die physikalischen Eigenschaften der wirkstoffhaltigen Teile des Systems nachteilig beeinflußt und/oder der Wirkstoff sehr teuer ist. Im Falle des Fentanyl treffen beide Gründe zu, wobei zusätzlich noch in Betracht zu ziehen ist, daß Fentanyl und seine Derivate zu den Betäubungsmitteln zählen und es allein schon deshalb wünschenswert ist, möglichst wenig Wirkstoff in das TTS einzuarbeiten bzw. die Wirkstoffausnutzung, d.h. das Verhältnis von während der Tragezeit des TTS abgegebenem Wirkstoff zum Gehalt des ungetragenen TTS möglichst groß zu gestalten.

Unter dem Gesichtspunkt sollte die Sättigungslöslichkeit der wirkstoffhaltigen Schichten für ein Dreitage-TTS nicht unter 3 Gewichtsprozenten und nicht über 20 Gewichtsprozenten liegen. Bei höheren Sättigungslöslichkeiten wird selbst bei einer hohen spezifischen Permeationsrate die Wirkstoffausnutzung zu schlecht, und das TTS ist aus kommerziellen Gründen wegen des teuren Wirkstoffs nicht gut verkäuflich. Bevorzugt liegt aus diesen Gründen die Sättigungslöslichkeit zwischen 4 und 12 und besonders bevorzugt zwischen 5 und 10 Gewichtsprozenten.

Die Sättigungslöslichkeit von Fentanyl und seinen Analogen kann zusätzlich reduziert werden durch den Zusatz von Substanzen, die keine guten Löseeigenschaften für den Wirkstoff haben. Solche Substanzen sind z.B. flüssige Kohlenwassestoffe wie Dioctylcyclohexan, flüssiges Paraffin, Kohlenwasserstoffharze wie Polyterpene, insbesondere Polypinen oder polare Substanzen wie Glycerin, Di- und Triglycerin oder Polyethylenglykole, z. B. mit einem Molgewicht von 200 bis 1000. Diese Substanzen können mit dem Polyacrylatkleber eine homogene Mischung bilden oder aber als eine gesonderte Phase darin enthalten sein. Speziell Glycerin und seine Derivate liegen schon bei geringen Konzentrationen in der Matrix als gesonderte Phase, z. B. in Form von Tröpfchen vor. Durch die Zugabe solcher Substanzen kann insbesondere auch die höhere Sättigungslöslichkeit in Klebern mit freien Hydroxylgruppen kompensiert werden.

Tabelle 2 enthält einige Angaben zu den Sättigungslöslichkeiten von Fentanyl in einigen dieser Substanzen.

**Tabelle 2: Sättigungslöslichkeiten von Fentanyl in löslichkeitsvermindemden Zusätzen**

| **Substanz** | **Sättigungslöslichkeit [Gew.-%]** |
|---|---|
| Polyethylenglykol 400 | 7,5 |
| Glycerin | < 1,5 |
| Diglycerin | < 1,5 |
| Dioctylcyclohexan | < 1,9 |
| Paraffin, flüssig | < 1,5 |

Der Einfluß von solchen Zusätzen läßt sich anhand von vergleichenden Permeationsstudien erkennen.

In Tabelle 3 sind die Ergebnisse von Permeationsstudien mit Matrices auf Basis eines neutralen Polyacrylatklebers mit freien Hydroxylgruppen mit und ohne solche Zusätze sowie eines Polyacrylatklebers ohne andere freie funktionelle Gruppen gegenübergestellt. Alle Formulierungen enthalten Fentanyl in einer Konzentration von 5 Gew. %.

**Tabelle 3. Vergleichende Permeationsstudien mit Formulierungen mit und ohne löslichkeitsvermindernde Zusätze**

| Formulierung | Kumulierte Menge an permeiertem Wirkstoff [µg/cm²] Mittelwert von n = 3* | | | | |
|---|---|---|---|---|---|
| | 4 h | 8 h | 24 h | 48 h | 72 h |
| | | | | | |
| 2 | 0,00 | 0,23 | 7,89 | 32,82 | 64,17 |
| | | | | | |
| 3 | 0,798 | 4,46 | 29,6 | 68,9 | 103,1 |
| | | | | | |
| 4 | 0,805 | 4,87 | 32,6 | 74,7 | 113,2 |

| | | | | | |
|---|---|---|---|---|---|
| • Haut: menschliche Epidermis, weibliche Brusthaut | | | | | |

| | | |
|---|---|---|
| Formul. 2: | 5 Gew. % Fentanyl in einem neutralen Polyacrylatkleber mit freien 5,2 % Hydroxylgruppen | |
| Formul. 3: | Fentanyl | 5,0 % |
| | Polyacrylatkleber, neutral mit freien 5,2 % | 55,0 % |
| | Hydroxylgruppen Polypinen | 15,0 % |
| | Glycerin | 10,0 % |
| | Dioctylcyclohexan | 15,0 % |
| Formul. 4: | 5 Gew. % Fentanyl in einem Polyacrylatkleber ohne freie funktionelle Gruppen | |

Die Ergebnisse der Permeationsstudie zeigen, daß die Permeationsrate durch den Zusatz von die Löslichkeit des Wirkstoffs in der Matrix reduzierenden Substanzen signifikant verbessert werden kann. In etwa die gleichen Ergebnisse erreicht man durch die Verwendung eines Klebers ohne freie funktionelle Gruppen, der auch ohne Zusätze über eine geringe Lösekapazität für den Wirkstoff verfügt.

Aus den Permeationsdaten lassen sich für verschiedene TTS-Stärken die jeweiligen TTS-Größen berechnen. Die Ergebnisse sind in Tabelle 4 gelistet.

**Tabelle 4: Aus Permeationsdaten errechnete TTS-Größen**

| **Abgaberate** | **berechnete Flächengrößen [cm²]** | | | | |
|---|---|---|---|---|---|
| | **Form. 1** | **Form. 2 *** | **Form. 2** ** | **Form. 3** | **Form. 4** |
| **25 µm/h** | 513 | 63,7 | 28,1 | 17,45 | 15,9 |
| **50 µm/h** | 1026 | 127,4 | 56,2 | 34,9 | 31,8 |
| **75 µm/h** | 1539 | 191,1 | 84,3 | 52,35 | 47,7 |
| **100 µm/h** | 2052 | 254,8 | 112,4 | 69,8 | 63,6 |

| | | | | | |
|---|---|---|---|---|---|
| * berechnet auf Basis der Permeationsdaten aus Tabelle 1 ** berechnet auf Basis der Permeationsdaten aus Tabelle 2 | | | | | |

Das Ergebnis der Berechnung zeigt, daß carboxylgruppenhaltige Kleber bei einer Fentanylkonzentration von 5 % auch bei der niedrigsten Dosierung zu für den praktischen Gebrauch zu großen TTS führen. Bei den hydroxylgruppenhaltigen Klebern berechnen sich zwar auch recht große TTS, allerdings besteht hier durch die Erhöhung der Fentanylkonzentration die Möglichkeit, bei nicht zu hohen Konzentrationen, d.h. höchstens 20 %, zu TTS mit einer für den praktischen Gebrauch geeigneten Größe zu kommen. Vereinfacht kann dabei angenommen werden, daß die thermodynamische Aktivität und damit auch die Permeationsraten linear von der Konzentration abhängen, solange der Wirkstoff vollständig gelöst vorliegt.

Durch Verwendung von die Löslichkeit senkenden Hilfsstoffen in Formulierungen mit hydroxylgruppenhaltigen Polyacrylatklebern bzw. durch die Verwendung von Polyacrylatklebern ohne freie funktionelle Gruppen erhält man schon bei einer Fentanylkonzentration von 5,0 % TTS, die selbst in der höchsten Dosierung von 100 µg/h eine akzeptable Größe aufweisen. Natürlich bietet sich auch hier die Möglichkeit, durch eine Erhöhung der Fentanylkonzentration die Systemfläche weiter zu verkleinern.

Fentanyl und seine Derivate haben, wie schon eingangs erwähnt, einen engen therapeutischen Index. Dies bedeutet, daß zur Wirkung einerseits ein gewisser Schwellenwert, der bezüglich der Plasmakonzentration überschritten sein muß, andererseits bei höheren Konzentrationen schnell unakzeptable Nebenwirkungen auftreten. Es ist deshalb vorteilhaft, wenn das System zusätzlich eine Steuermembran enthält und damit den Wirkstofffluß durch die Haut unabhängig von der individuellen Hautbeschaffenheit auf einen Maximalwert begrenzt. Solche Membranen bestehen bevorzugt aus einem Copolymer aus Ethylen und Vinylacetat (EVA-Polymer) oder sind mikroporöse Folien auf der Basis von Polyethylen oder Polypropylen. Derartige Membranen gehören zum Stand der Technik. Im Falle der EVA-Polymere hängt die Wirkstoffdurchlässigkeit von dem Anteil des Vinylacetats und der Dicke der Membran ab. Gebräuchlich sind Membranen mit einem VA-Gehalt zwischen 2 und 25 Gewichtsprozenten und einer Dicke zwischen 25 und 100 µm, vorzugsweise zwischen 40 und 100µm, wobei es bezüglich des Vinylacetat-Gehalts und der Dicke praktisch kaum Begrenzungen gibt. Für die jeweilige Formulierung müssen beide Parameter entsprechend gewählt werden, um die Begrenzung auf den gewünschten Maximalfluß aus dem TTS zu gewährleisten. Bei den mikroporösen Membranen erfolgt der Stofftransport nicht durch das Polymer, sondern lediglich durch die sich in diesen Membranen befindlichen Poren. Die Anzahl und Größe der Poren bestimmt dabei die maximale Abgaberate des TTS.

Üblicherweise sind solche Membranen mit einem Kleberfilm zur Befestigung des TTS auf der Haut versehen. Besonders geeignet für Fentanyl und seine Derivate sind Kleberfilme auf Basis von selbstklebenden Polyacrylaten oder selbstklebenden Polysiloxanen. Der Vorteil von Polysiloxanen ist dabei, daß der Wirkstoff in diesen Polymerem sehr schlecht löslich ist und deshalb die Wirkstoffbeladung des TTS durch die Verwendung eines zusätzlichen Kleberfilms nicht unnötig gesteigert werden muß. Derartige Kleberfilme können jedoch auch bei Systemen angewendet werden, die keine Membranen, aber Matrixschichten mit geringerer Klebkraft enthalten.

Wie bei jedem TTS gibt es natürlich auch hier die Möglichkeit, die Barriereeigenschaften des menschlichen Stratum Corneum durch den Einsatz von permeationsfördernden Stoffen zu reduzieren. Solche Substanzen sind z.B. Fettsäuren, Fettalkohole, Fettsäureester, Ester des Glycerins mit mittel- bzw. langkettigen Fettsäuren und Glycole wie 1,2-Propandiol. Es können dabei alle Substanzen eingesetzt werden, die physiologisch unbedenklich und mit dem Wirkstoff und den anderen Hilfsstoffen verträglich sind.

Zusammenfassend ist festzustellen, das Matrixsysteme im Sinne dieser Erfindung befriedigende bis gute Permeationsraten zeigen und auch die Herstellung von TTS mit einer akzeptablen Größe ermöglichen. Gleichzeitig ist eine Gefährdung des Patienten durch eine zu hohe Wirkstoffaufnahme infolge einer Undichtigkeit unmöglich. Insgesamt stellen damit Matrixsysteme auf Basis von Polyacrylatklebern im Sinne dieser Erfindung für Fentanyl und seine Analogen bezüglich der Patientensicherheit einen bedeutenden Fortschritt gegenüber dem bekannten Stand der Technik dar.

### Beispiel 1 (Formulierung 1, 2, 4)

Fentanyl (freie Base) wird in der Lösung des Klebers im Heptan/Ethylacetat gelöst. Die Menge an Fentanyl wird dabei so berechnet, daß sich, bezogen auf den Feststoffgehalt der Kleberlösung, eine Konzentration von 5,0 % ergibt. Die resultierende Masse wird mit einem Rakel auf eine silikonisierte Polyesterfolie vor Gebrauch zu entfernende Schutzschicht, in einer Dicke beschichtet, daß sich nach dem Entfernen der Lösemittel ein Gewicht der Beschichtung von ca. 80 g/ m² ergibt. Nach dem Entfernen der Lösemittel wird der getrocknete Film mit einer dünnen Polyesterfolie (Rückschicht des TTS) kaschiert, und aus dem Gesamtlaminat werden die fertigen TTS ausgestanzt.

### Beispiel 2 (Formulierung 3):

5,0 g Fentanyl, 15,0 g Polypinen, 10,0 g Glycerin, 15,0 g Dioctylcyclohexan und 110 g der Kleberlösung mit einem Feststofffgehalt von 50,0 % werden zusammengegeben und bis zum Auflösen des Fentanyls gerührt.
Die resultierende Masse wird mit einem Rakel auf eine silikonisierte Polyesterfolie (vor Gebrauch zu entfernende Schutzschicht) in einer Dicke beschichtet, daß sich nach dem Entfernen der Lösemittel ein Gewicht der Beschichtung von ca. 80 g/ m² ergibt. Nach dem Entfernen der Lösemittel wird der getrocknete Film mit einer dünnen Polyesterfolie (Rückschicht des TTS) kaschiert und aus dem Gesamtlaminat werden die fertigen TTS ausgestanzt.

Die folgenden Seiten 12 und 13 enthalten bevorzugte Ausführungsformen der Erfindung; der auf den Seiten 12 und 13 benutzte Begriff "Patentansprüche" meint diese Ausführungsformen der Erfindung.
1. Transdermales Therapeutisches System (TTS) bestehend aus einer wirkstoffundurchlässigen Rückschicht, zumindest einer Fentanyl oder einen fentanylanalogen Wirkstoff enthaltenden Matrixschicht auf Basis von Polyacrylat und einer vor Gebrauch zu entfernenden Schutzschicht, dadurch gekennzeichnet, daß das Polyacrylat selbstklebend und frei von Carboxylgruppen ist, für Fentanyl eine Sättigungslöslichkeit zwischen 3 und 20 Gewichtsprozenten, bevorzugt eine Sättigungslöslichkeit zwischen 4 und 12 und besonders bevorzugt eine Sättigungslöslichkeit zwischen 5 und 10 Gewichtsprozenten aufweist, und daß die wirkstoffhaltigen Schichten mindestens 80 Gewichtsprozent des eingearbeiteten Wirkstoffs in molekulardispers gelöster Form enthalten.
2. TTS gemäß Anspruch 1, dadurch gekennzeichnet, daß das Polyacrylatpolymer über keine freien funktionellen Gruppen verfügt und lediglich aus Monomeren der Acryl- bzw. Methacrylsäureester und gegebenenfalls zusätzlich aus anderen polymerisierbaren Vinylverbindungen ohne freie funktionelle Gruppen in Mengen von bis zu 50 Gew.-%, insbesondere Vinylacetat aufgebaut ist.
3. TTS gemäß Anspruch 1, dadurch gekennzeichnet, daß das dem Polyacrylat zugrunde liegende Monomerengemisch bis zu 20 Gew.-% Monomere mit freien funktionellen Gruppen in Form von 2-Hydroxyethylacrylat und/oder -methacrylat enthält.
4. TTS gemäß einem oder mehreren der Ansprüche 1-3, dadurch gekennzeichnet, daß es als weitere Schicht zusätzlich eine Steuermembran enthält.
5. TTS gemäß Anspruch 4, dadurch gekennzeichnet, daß es zusätzlich eine sich hautwärts auf der Membran befindende selbstklebende Schicht zur Befestigung auf der Haut enthält.
6. TTS gemäß Anspruch 4 oder 5, dadurch gekennzeichnet, daß die Steuermembran aus einem Ethylen-Vinylacetat-Copolymer, zweckmäßig mit einem Vinylacetatanteil von bis zu 25 Gew.-%, oder einer mikroporösen Folie auf Basis von Polyethylen oder Polypropylen besteht und zweckmäßig eine Dicke zwischen 25 und 100, vorzugsweise zwischen 40 und 100 µm aufweist.
7. TTS gemäß einem oder mehreren der Ansprüche 1-6, dadurch gekennzeichnet, daß die wirkstoffhaltgen Schichten zusätzlich die Permeationsrate durch menschliche Haut verbessernde Substanzen enthalten, insbesondere Glykole und/oder solche, die zur Gruppe der Fettsäuren, Fettsäureester, Fettalkohole oder Glycerinester gehören.
8. TTS gemäß einem oder mehreren der Ansprüche 1-7, dadurch gekennzeichnet, daß die wirkstoffhaltigen Schichten Substanzen enthalten, die die Löslichkeit des Wirkstoffs in diesen Schichten erniedrigen.
9. TTS gemäß Anspruch 8, dadurch gekennzeichnet, daß die die Löslichkeit erniedrigenden Substanzen bei Raumtemperatur flüssige Kohlenwasserstoffe wie Dioctylcyclohexan oder flüssiges Paraffin, Kohlenwasserstoffharze wie Polypinenharze oder Polyethylenglykol oder Glycerin sind.

## Patentansprüche

1. Eine für den Einsatz in einem Transdermalen Therapeutischen System (TTS) geeignete, Fentanyl oder einen fentanylanalogen Wirkstoff enthaltende Matrix-Zusammensetzung auf Basis von Polyacrylat, **dadurch gekennzeichnet, dass** das Polyacrylat selbstklebend, frei von Carboxylgruppen ist, lediglich aus monomeren Estern von Alkoholen mit 1 bis 8 C-Atomen und Acrylsäure und/oder Methacrylsäure, wobei diese Ester gegebenenfalls eine Hydroxylgruppe enthalten, und gegebenenfalls aus bis zu 50 Gew.-% Vinylacetat hergestellt wird, für den Wirkstoff eine Sättigungslöslichkeit zwischen 3 und 20 Gewichtsprozenten aufweist, und dass die wirkstoffhaltigen Schichten mindestens 5 Gewichtsprozent des eingearbeiteten Wirkstoffs enthalten, von dem mindestens 80 Gewichtsprozent in molekulardispers gelöster Form vorliegen.

2. Matrix-Zusammensetzung gemäß Anspruch 1, **dadurch gekennzeichnet, dass** das Polyacrylat für den Wirkstoff eine Sättigungslöslichkeit zwischen 4 und 12 Gewichtsprozenten aufweist.

3. Matrix-Zusammensetzung gemäß Anspruch 2, **dadurch gekennzeichnet, dass** das Polyacrylat für den Wirkstoff eine Sättigungslöslichkeit zwischen 5 und 10 Gewichtsprozenten aufweist.

4. Matrix-Zusammensetzung gemäß Anspruch 1, **dadurch gekennzeichnet, dass** das Polyacrylat keine Hydroxylgruppen enthält.

5. Matrix-Zusammensetzung gemäß einem der Ansprüche 1 bis 3, **dadurch gekennzeichnet, dass** das dem Polyacrylat zugrunde liegende Monomerengemisch bis zu 20 Gew.-% 2-Hydroxyethylacrylat und/oder -methacrylat als Monomere mit freien Hydroxylgruppen enthält.

6. Matrix-Zusammensetzung gemäß einem der Ansprüche 1 bis 3 und 5, **dadurch gekennzeichnet, dass** die Polymerketten im Polyacrylat mit freien Hydroxylgruppen durch mehrwertige Kationen oder reaktive Substanzen vernetzt sind.

7. Matrix-Zusammensetzung gemäß Anspruch 6, **dadurch gekennzeichnet, dass** die Vernetzung durch Al³⁺, Ti⁴⁺ oder Melamin erfolgt ist.

8. Matrix-Zusammensetzung gemäß einem der Ansprüche 1 bis 7, **dadurch gekennzeichnet, dass** sie zusätzlich die Permeationsrate durch menschliche Haut verbessernde Substanzen enthält, insbesondere Glykole und/oder solche, die zur Gruppe der Fettsäuren, Fettsäureester, Fettalkohole oder Glycerinester gehören.

9. Matrix-Zusammensetzung gemäß einem der Ansprüche 1 bis 8, **dadurch gekennzeichnet, dass** sie Substanzen enthält, die die Löslichkeit des Wirkstoffs in der Matrix erniedrigen.

10. Matrix-Zusammensetzung gemäß Anspruch 9, **dadurch gekennzeichnet, dass** die die Löslichkeit erniedrigenden Substanzen bei Raumtemperatur flüssige Kohlenwasserstoffe, insbesondere Dioctylcyclohexan oder flüssiges Paraffin, Kohlenwasserstoffharze, insbesondere Polypinenharze oder Polyethylenglykol, oder Glycerin, sind.

11. Verwendung einer Matrix-Zusammensetzung gemäß einem der Ansprüche 1 bis 10 als selbstklebende und Wirkstoff enthaltende Schicht in einem Transdermalen Therapeutischen System (TTS), das außerdem eine wirkstoffundurchlässige Rückschicht und eine vor Gebrauch zu entfernende Schutzschicht enthält.

12. Verwendung gemäß Anspruch 11, **dadurch gekennzeichnet, dass** das TTS als weitere Schicht zusätzlich eine Steuermembran enthält.

13. Verwendung gemäß Anspruch 12, **dadurch gekennzeichnet, dass** das TTS zusätzlich eine sich hautwärts auf der Steuermembran befindende selbstklebende Schicht zur Befestigung auf der Haut enthält.

14. Verwendung einer Matrixzusammensetzung aus einem Polyacrylat und Fentanyl oder einem Fentanylanalogen als Wirkstoff, wobei das Polyacrylat selbstklebend und frei von Carboxylgruppen ist, für den Wirkstoff eine Sättigungslöslichkeit zwischen 4 und 12 und besonders bevorzugt eine Sättigungslöslichkeit zwischen 5 und 10 Gewichtsprozenten aufweist, und daß die wirkstoffhaltigen Schichten mindestens 80 Gewichtsprozent des eingearbeiteten Wirkstoffs in molekulardispers gelöster Form enthalten, als selbstklebende und Wirkstoff enthaltende Schicht in einem Transdermalen Therapeutischen System (TTS), das außerdem eine wirkstoffundurchlässige Rückschicht und eine vor Gebrauch zu entfernende Schutzschicht enthält.

15. Verwendung eines Polyacrylates, das selbstklebend und frei von Carboxylgruppen ist, für Fentanyl oder einen fentanylanalogen Wirkstoff das eine Sättigungslöslichkeit zwischen 3 und 20 Gewichtsprozenten aufweist zur Herstellung der wirkstoffhaltigen Schicht eines Transdermalen Therapeutischen Systems (TTS), bei dem es sich um ein Drei-Tage-TTS handelt.
